# EUROPÄISCHE PATENTSCHRIFT

(11) **EP 2 681 813 B1**
(45) Veröffentlichungstag und Bekanntmachung des Hinweises auf die Patenterteilung: **01.04.2015**
(21) Anmeldenummer: 12704223.2
(22) Anmeldetag: 23.01.2012
(51) Int. Cl.: H01R 13/518, H01R 101/00

(54) **MODULARER STECKVERBINDER**
MODULAR PLUG CONNECTOR
CONNECTEUR ENFICHABLE MODULAIRE

(30) Priorität: 03.03.2011 DE 102011001064
(43) Veröffentlichungstag der Anmeldung: 08.01.2014
(73) Patentinhaber: Harting Electric GmbH & Co. KG, 32339 Espelkamp (DE)
(72) Erfinder: SCHLEGEL, Bernard, 32369 Rahden (DE)
(86) Internationale Anmeldenummer: PCT/DE2012/100016
(87) Internationale Veröffentlichungsnummer: WO 2012/116692

(56) Entgegenhaltungen:
- CH-B1- 697 606
- DE-C1- 3 627 899
- DE-U1- 29 701 158
- GB-A- 1 536 082
- US-A- 3 824 553

## Beschreibung

Die Erfindung betrifft einen Steckverbinder nach dem Oberbegriff des Anspruchs 1.

Derartige Steckverbinder werden benötigt, um Kontakt zwischen den einzelnen Leitern eines angeschlossenen Kabels und den Kontakten eines Gegensteckers oder einer Buchse eines Gerätes herzustellen. Bei den einzelnen Leiter des angeschlossenen Kabels kann es sich um metallische Leiter, aber auch um Glasfasern oder vergleichbares Material handeln.

### Stand der Technik

Die US 3 824 553 A, DE 36 27 899 C1 und die DE 80 10 524 U1 zeigen jeweils Steckverbindersysteme, bei denen die einzelnen Steckverbindergehäuse Mittel aufweisen, mit denen sie untereinander verbindbar sind. Weiterhin weisen sie auch Fixiermittel auf, mit denen sie einzeln oder im Verbund an Montageschienen befestigbar sind.

Die DE 20 2008 006 934 U1 zeigt einen Steckverbinder für Lichtwellenleiter. Die Gehäusekörper tragen jeweils nur ein Kontaktierungselement und können aneinandergereiht werden, indem am Gehäusekörper angeformte Stege in dazu passende Ausnehmungen eines benachbarten Gehäusekörpers geführt werden.

Übersteigen die aneinandergereihten Steckerverbinder eine gewisse Anzahl, wird die Steckverbinderreihe unstabil, so dass eine Basis, wie beispielsweise eine Montageschiene (Hutschiene) vorteilhaft wäre. Die Gehäusekörper der DE 20 2008 006 934 U1 haben allerdings keinerlei geeignete Fixierungsmittel für die Montage auf einer solchen Montageschiene. Daher können solche Steckverbinder beispielsweise nicht in Schaltschränken eingesetzt werden.

### Aufgabenstellung

Die Aufgabe der Erfindung besteht darin, einen Steckverbinder vorzuschlagen, der einfach herstellbar und vielseitig einsetzbar ist.

Die Aufgabe wird durch die kennzeichnenden Merkmale des Anspruchs 1 gelöst.

Vorteilhafte Ausgestaltungen der Erfindung sind in den Unteransprüchen angegeben.

Der erfindungsgemäße Steckverbinder ist von einem Gehäusekörper umgeben.

Vorteilhafterweise ist der Gehäusekörper einteilig ausgestaltet. Dies kann durch die bekannten Kunststoffspritzgussverfahren - im Falle eines Kunststoffgehäuses - oder durch bekannte Zinkdruckgussverfahren - im Falle eines metallischen Gehäuses - realisiert werden.

Es ist auch möglich den Gehäusekörper aus einem Kompositwerkstoff, beispielsweise aus einer Kombination von Metall und Kunststoff, zu gestalten.

Innerhalb des Gehäusekörpers ist ein Haltekäfig verliersicher verrastet.

Der Haltekäfig ist im Wesentlichen als Hohlzylinder ausgestaltet. An einem Ende weist der Haltekäfig zwei in axialer Richtung weisende Arme auf, die ein Kontaktierungselement verliersicher umgreifen. Am anderen Ende umgreift der Haltekäfig den Kabelmantel eines an den Steckverbinder anzuschließenden Kabels. Ein Leiter des Kabels ist mit dem Kontaktierungselement beispielsweise über eine Crimpverbindung kontaktiert.

An der Außenseite des Gehäusekörpers sind beidseitig Kuppelelemente vorgesehen, die es ermöglichen zwei oder mehr Steckverbinder mit gleichartigem Gehäusekörper zusammen zu kuppeln.

Vorzugsweise ist das eine Kuppelelement als bogenförmige Nut und das andere Kuppelelement als zylinderförmiger Stift ausgebildet. Der zylinderförmige Stift des einen Gehäusekörpers ist dazu ausgelegt in die bogenförmige Nut des zu kuppelnden gleichartigen Gehäusekörpers eingeführt zu werden. So können mehrere Steckverbinder mit gleichartigem Gehäusekörper aneinandergereiht werden.

Bei der Aneinanderreihung brauchen nur die Gehäusekörper gleichartig ausgeführt zu sein. Das Innenleben der Steckverbinder kann durchaus unterschiedlich sein. So können beispielsweise mehrpolige elektrische Steckverbinder mit einpoligen Lichtwellenleiter-Steckverbindern kombiniert werden. Dadurch kann eine hohe Modularität einer Steckverbinderanordnung erreicht werden. Eine Steckverbinderanordnung wird hier auch als System von Steckverbindern bezeichnet.

Außen am Gehäusekörper sind außerdem Fixiermittel angeformt, die es ermöglichen den Steckverbinder auf einer Montageschiene, beispielsweise einer Hutschiene, zu fixieren.

Die Steckverbinder können untereinander über die Kuppelelemente miteinander gekoppelt werden und außerdem auf einer Montageschiene verliersicher fixiert werden.

Die Fixiermittel bestehen aus einer Kombination von Fixiernasen und Federstiften. Die Fixiernasen stehen in axialer Richtung ab und greifen in einen Kragen der Montageschiene ein. Die Fixierstifte sind federnd ausgeführt und können beim Verrasten auf der Montageschiene zunächst ausweichen, bevor sie hinter einen weiteren Kragen der Montageschiene einrasten.

Der Steckverbinder wird durch eine Kabelverschraubung komplettiert, die die Kabelzugentlastung und die Abdichtung des Gehäusekörpers gegen Medien wie Staub und Wasser gewährleistet.

Wenn eine gewünschte Anzahl von Steckverbindern auf einer Montageschiene aufgereiht ist, sprich man auch von einem System von Steckverbindern.

Um zwei Systems von Steckverbindern miteinander zu kontaktieren, müssen die Montageschienen auf denen sich jeweils die Systeme von Steckverbindern befinden zusammengeführt werden. Dies wird über eine Verriegelungsvorrichtung realisiert, die die einzelnen Montageschienen verliersicher miteinander verbindet, so dass auch die gegenüberliegenden Steckverbinder miteinander kontaktiert werden.

Die Verriegelungsvorrichtung besteht aus einem passiven Verriegelungsteil und einem aktiven Verriegelungsteil. Das aktive Verriegelungsteil trägt die Verriegelungselemente, mit denen das passive Verriegelungsteil an das aktive Verriegelungsteil angebunden wird.

Beim Verriegeln des aktiven mit dem passiven Verriegelungsteil, werden die Montageschienen der Systeme von Steckverbindern zueinander geführt und miteinander verbunden. Außerdem werden die einzelnen, gegenüberliegenden Steckverbinder miteinander kontaktiert.

Vorteilhafterweise bilden die Verriegelungselemente des passiven Verriegelungsteils eine Kniehebelverriegelung. Dadurch wird eine besonders stabile Verriegelung erreicht.

Um das Zusammenführen der miteinander zu verbindenden Montageschienen zu erleichtern, weist die Verriegelungsvorrichtung eine Führung auf. Die Führung besteht aus einem Bolzen am aktiven Verriegelungsteil, der beim Verriegelungsvorgang in eine dafür vorgesehene Öffnung im passiven Verriegelungsteil geführt wird.

Das passive Verrieglungsteil umfasst beidseitig Fixiermittel mit welchen eine Montageschiene fixierbar ist. So können zwei Montageschienen übereinander - also horizontal beabstandet - statisch stabil angeordnet werden. Durch das Aneinanderkoppeln des passiven mit dem aktiven Verriegelungsteils können dann kontaktierte Systeme von Steckverbindern horizontal beabstandet und statisch stabil angeordnet werden. Über die Verriegelungsvorrichtung können beliebig viele Systeme von Steckverbindern übereiander angeordnet und so genannte Steckverbinder-Arrays gebildet werden.

### Ausführungsbeispiel

Ein Ausführungsbeispiel der Erfindung ist in den Zeichnungen dargestellt und wird im Folgenden näher erläutert. Es zeigen:
- Fig. 1: eine perspektivische Darstellung eines Steckverbinders,
- Fig. 2: eine weitere perspektivische Darstellung des Steckverbinders,
- Fig. 3: eine perspektivische Darstellung des Steckverbinders ohne Grundkörper,
- Fig. 4: eine perspektivische Darstellung eines Kontaktierungselements,
- Fig. 5: eine Seitenansicht des auf einer Montageschiene montierten Steckverbinders,
- Fig. 6: eine perspektivische Darstellung zweier Steckverbinder beim Koppelvorgang,
- Fig. 7: eine perspektivische Ansicht zweier miteinander verbundenen Systeme von Steckverbinder und
- Fig. 8: eine Seitenansicht der Verriegelungsvorrichtung.

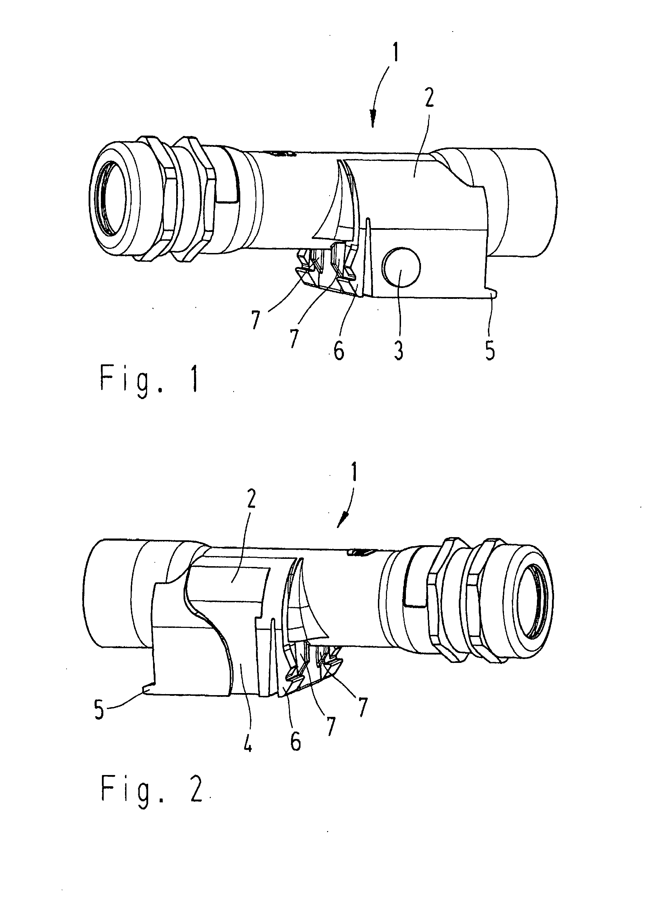

Die Figuren 1 und 2 zeigen perspektivische Darstellungen eines Ausführungsbeispiels des erfindungsgemäßen Steckverbinders. Die Erfindung ist aber nicht auf das hier gezeigte Ausführungsbeispiel beschränkt.

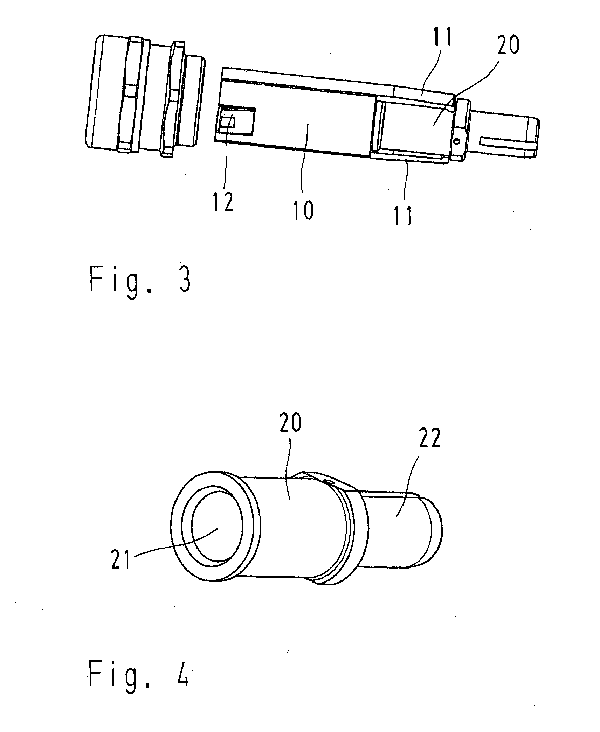

Der Steckverbinder 1 wird vom einem Grundkörper 2 umschlossen. Innerhalb des hohlen Grundkörpers 2 ist ein Haltekäfig 10 verliersicher verrastet. Die Figur 3 zeigt den Steckverbinder 1 ohne Grundkörper 2. Der Haltekäfig 10 weist an einem Ende Verrastelemente 12 auf, die in dazu passende Hinterschnitte (nicht gezeigt) innerhalb des Grundkörpers eingreifen und den Haltekäfig 10 so im Grundkörper 2 fixieren. An diesem Ende durchdringt auch der Kabelmantel des anzuschließenden Kabels (nicht gezeigt) den Haltekäfig.

An einem anderen Ende weist der Haltekäfig Arme 11 auf, die das Kontaktierungselement 20 umgreifen. Dadurch wird das Kontaktierungselement 20 im Grundkörper 2 entlang der axialen Symmetrieachse fixiert.

Wie bereits oben erwähnt, wird das gegenüberliegende Ende des Haltekäfigs 10 vom Kabelmantel des anzuschließenden Kabels durchdrungen. Der Leiter des anzuschließenden Kabels ist mit dem Kontaktierungselement 20 vercrimpt.

Die Figur 4 zeigt die perspektivische Darstellung des Kontaktierungselements 20. Einseitig weist das Kontaktierungselement 20 eine Crimpöffnung 21 zur Aufnahme des Leiters des anzuschließenden Kabels auf. Die andere Seite des Kontaktierungselements 20 ist in diesem Ausführungsbeispiel als Stiftkontakt 22 ausgeführt. Es kann aber auch ein Buchsenkontakt vorgesehen sein.

Die Erfindung ist nicht auf Einkontaktsteckverbinder beschränkt. Das Kontaktierungselement 20 kann auch mehrere Crimpöffnungen 21 und Kontakte 22 zum anschließen mehradriger Kabel aufweisen.

Der Grundkörper 2 umfasst an einer Seite einen zylinderförmigen Stift 3 und gegenüberliegend eine bogenförmige Nut 4. Der Stift 3 eines Steckverbinder 1 ist dazu geeignet, in die Nut 4 eines weiteren Steckverbinders 1 eingeführt zu werden. So werden mehrere Steckverbinder 1 aneinandergereiht beziehungsweise miteinander gekoppelt. Ist eine gewünschte Anzahl von Steckverbindern erreicht, spricht man von einem System von Steckverbindern 1, 1'.

Am Gehäusekörper 2 sind außerdem Fixiernasen 5 und Federstifte 6 vorgesehen, die zusammen das reversible Fixieren des Steckverbinders 1 auf einer Montageschiene 30 ermöglichen. Die Fixiernasen 5 greifen in den Hinterschnitt 31 der Montageschiene 30 ein. Beim Herunterklappen des Steckverbinders 1 in Richtung der Montageschiene 30, werden die Federstifte von einem angeschrägten Kranz 33 zunächst zurückgebogen, um dann in einen weiteren Hinterschnitt 32 der Montageschiene 30 einzugreifen.

Der Gehäusekörper 2 umfasst weiterhin Abstützkonturen 7, die verhindern, dass bei einer Bewegung der Steckverbinders 1 auf der Montageschiene 30 in Steckrichtung, die daraus resultierende Kraft nicht vollständig von den Federstiften aufgefangen werden muss.

Der Steckverbinder 1 ist mit einer Kabelverschraubung 40 versehen, die aus dem Stand der Technik allgemein bekannt ist. Daher wird hier nicht näher auf die Kabelverschraubung eingegangen. Die Kabelverschraubung 40 dient zur Zugentlastung des anzuschließenden Kabels und zur Abdichtung des Grundkörpers 2 gegen Medien wie Staub und Wasser.

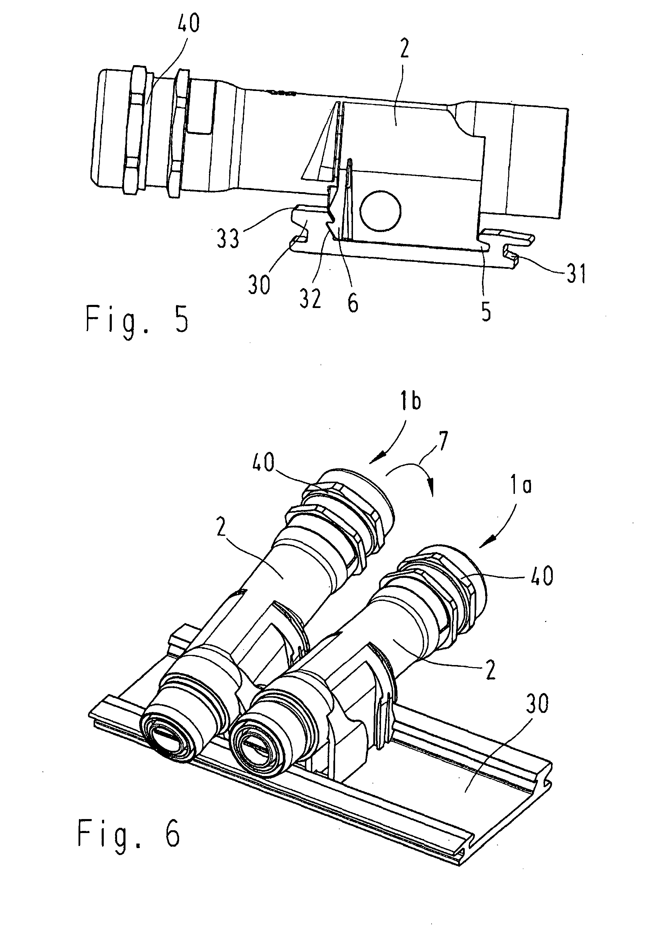

Die Figur 6 zeigt den Koppelvorgang zweier Steckverbinder auf der Montageschiene 30. Ein Steckverbinder 1a ist hier bereits auf der Montageschiene fixiert. Die Fixiernasen 5 des daran zu koppelnden Steckverbinders 1 b werden zunächst in den Hinterschnitt 31 eingelegt. Dann kann der Steckverbinder 1 b in Richtung des Pfeils 7 zur Montageschiene 30 hin bewegt werden. Die bogenförmige Nut des Steckverbinders 1 b greift hinter den zylinderförmigen Stift 3 des Steckverbinders 1a. Aufgrund der Bogenform der Nut 4, kann der Steckverbinder 1 b in Richtung des Pfeils 7 geführt werden. Anschließend rasten die Federstifte wie oben bereits beschrieben in den Hinterschnitt 32 der Montageschiene 30 ein. Die Steckverbinder 1a und 1b sind sowohl miteinander gekoppelt als auch auf der Montageschiene 30 fixiert.

Beim Abkoppeln eines Steckverbinders aus einem modularem System von Steckverbindern, wird genau umgekehrt verfahren. Die Federstifte 6 müssen zunächst händisch aus dem Hinterschnitt 32 gedrückt werden. Anschließend kann der Steckverbinder entgegen der Pfeilrichtung 7 vom benachbarten Steckverbinder und der Montageschiene 30 gelöst werden.

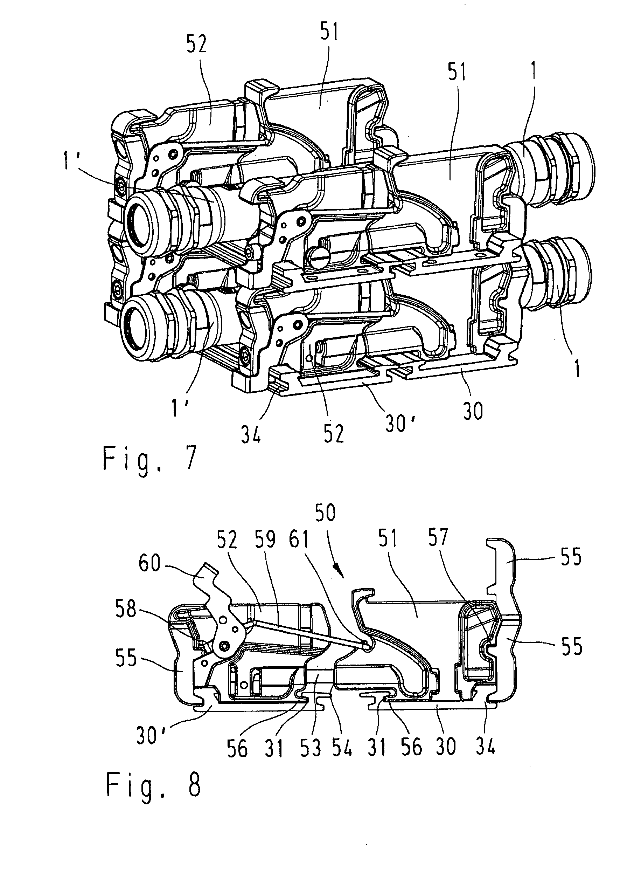

Die Figur 8 zeigt die Seitenansicht einer Verriegelungsvorrichtung 50. Die Verriegelungsvorrichtung 50 besteht aus einem passiven Verriegelungsteil 51 und einem aktiven Verriegelungsteil 52.

Sowohl das aktive 52 wie auch das passive Verriegelungsteil 51 weisen Nasen 56, die in die Hinterschnitte 31 der Montageschiene 30 einführbar sind. Über einen Verbinder 55 können beide Verriegelungsteile 51, 52 auf der jeweiligen Montageschiene 30, 30' fixiert werden. Der Verbinder 55 greift dabei gleichzeitig in eine Kerbe 57, 58 des Verbindungsteils 51, 52 und in eine hintere Nut 34 der Montageschiene 30 ein.

Um die Verriegelungsteile 51, 52 leicht zusammenführen zu können, ist eine Führung vorgesehen. Das passive Verriegelungsteil 51 umfasst eine Öffnung 54, in welche ein Bolzen 53 des aktiven Verriegelungsteils 52 einführbar ist.

Die Verriegelung beider Verriegelungsteile 51, 52 wird über eine am aktiven Verriegelungsteil 52 angebrachte Spannfeder 59 realisiert, die mit einem Spannhebel 60 (ebenfalls am aktiven Verriegelungsteil 52 angebracht) verbunden ist. Die Spannfeder 59 wird über eine Spannnase 61 des passiven Verriegelungsteiles 51 gelegt. Durch Betätigung des Spannhebels 60 werden die beiden Verriegelungsteile 51, 52 zusammengeführt und miteinander reversibel verbunden. Die Kombination aus Spannnase 61, Spannfeder 59 und Spannhebel 60 arbeitet nach dem Kniehebelprinzip und bewirkt dadurch gleichzeitig eine besonders stabile Koppelung der Verriegelungsteile 51, 52 und der miteinander kontaktierten Steckverbinder 1, 1'. Außerdem unterstützt das Kniehebelprinzip den Entriegelungsvorgang.

Die Figur 7 zeigt mehrere Ebenen von Systemen von Steckverbindern 1, 1'. Zwei gegenüberliegende Systeme von Steckverbindern 1, 1' sind über die Verriegelungsvorrichtung 50 miteinander kontaktiert. Auf die Verriegelungsvorrichtung 50 kann beidseitig eine Montageschiene 30, 30' fixiert werden. Dadurch können mehrere Systeme von Steckverbindern 1, 1' in einem gleichen Abstand übereinander angeordnet werden.

**Modularer Steckverbinder**

| | | | |
|---|---|---|---|
| 1 | **Steckverbinder** | 10 | **Haltekäfig** |
| 2 | Grundkörper | 11 | Arm |
| 3 | Zylinderförmiger Stift | 12 | Verrastelement |
| 4 | Bogenförmige Nut | | |
| 5 | Fixiernasen | | |
| 6 | Federstifte | **20** | **Kontaktierungselement** |
| 7 | Abstützkontur | 21 | Crimpöffnung |
| | | | |
| **30** | **Montageschiene** | **50** | **Verriegelungsvorrichtung** |
| 31 | Hinterschnitt | 51 | Passiver Verriegelungsteil |
| 32 | Hinterschnitt | 52 | Aktiver Verriegelungsteil |
| 33 | Kranz | 53 | Bolzen |
| 34 | Nut | 54 | Öffnung |
| | | 55 | Verbinder |
| **40** | **Kabelverschraubung** | 56 | Nase |
| | | 57 | Kerbe |
| | | 58 | Kerbe |
| | | 59 | Spannfeder |
| | | 60 | Spannhebel |
| | | 61 | Spannnase |

## Patentansprüche

1. Auf einer Montageschiene (30) fixierbarer Steckverbinder (1), 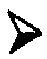 wobei außen am Gehäusekörper (2) des Steckverbinders (1) Fixiermittel (5, 6) zur Fixierung des Steckverbinders (1) auf einer Montageschiene (30) vorgesehen sind, 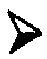
wobei innerhalb des Gehäusekörpers (2) des Steckverbinders (1) ein Haltekäfig (10) verrastet ist,
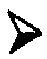 der Haltekäfig (10) einendig ein Kontaktierungselement (20), zur elektrischen Kontaktierung eines Leiters eines anzuschließenden Kabels und anderendig den Kabelmantel des anzuschließenden Kabels umgreift
**dadurch gekennzeichnet**,
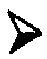 dass außen am Gehäusekörper (2) beidseitig Kuppelelemente (3, 4) zur reversiblen Verbindung mit einem weiteren Steckverbinder (1) mit gleichartigem Gehäusekörper (2) vorgesehen sind und
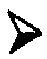 dass das eine Kuppelelement als bogenförmige Nut (4) und das andere Kuppelelement als zylinderförmiger Stift (3) ausgebildet ist.

2. Steckverbinder nach einem der vorstehenden Ansprüche
**dadurch gekennzeichnet, dass**
der Gehäusekörper (2) eine Kabelverschraubung (40), zur Kabelzugentlastung und Abdichtung aufweist.

3. Steckverbinder nach einem der vorstehenden Ansprüche
**dadurch gekennzeichnet, dass**
der Gehäusekörper (2) einstückig ausgeführt ist.

4. Steckverbinder nach einem der vorstehenden Ansprüche
**dadurch gekennzeichnet, dass**
die Fixiermittel aus einer Kombination von Fixiernasen (5) und Federstiften (6) gebildet sind.

## Claims

1. A plug connector (1) adapted to be fixed on a mounting rail (30),
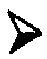 fixing means (5, 6) being provided on the outside of the housing body (2) of the plug connector (1) for fixing the plug connector (1) on a mounting rail (30),
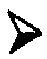 a holding cage (10) being locked in place inside the housing body (2) of the plug connector (1),
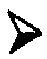 the holding cage (10) at one end engaging around a contacting element (20) for electrically contacting a conductor of a cable to be connected and at the other end engaging around the cable sheath of the cable to be connected,
**characterized in that**
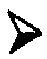 coupling elements (3, 4) are provided on the outside of the housing body (2) on both sides for a reversible connection with a further plug connector (1) having a housing body (2) of the same type, and
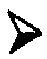 in that one coupling element is formed as an arcuate groove (4) and the other coupling element is formed as a cylindrical pin (3).

2. The plug connector according to any of the preceding claims,
**characterized in that**
the housing body (2) includes a screwed cable gland (40) for a cable strain relief and sealing.

3. The plug connector according to any of the preceding claims,
**characterized in that**
the housing body (2) is configured in one piece.

4. The plug connector according to any of the preceding claims,
**characterized in that**
the fixing means are formed of a combination of fixing noses (5) and spring pins (6).

## Revendications

1. Connecteur enfichable (1) apte à être fixé sur un rail de montage (30), 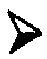
des moyens de fixation (5, 6) pour la fixation du connecteur enfichable (1) sur un rail de montage (30) étant prévus à l'extérieur sur le corps de boîtier (2) du connecteur enfichable (1),
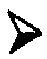 une cage de retenue (10) étant enclenchée à l'intérieur du corps de boîtier (2) du connecteur enfichable (1),
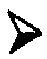 la cage de retenue (10) entourant à une extrémité un élément de contact (20) pour contacter électriquement un conducteur d'un câble à raccorder, et à l'autre extrémité la gaine de câble du câble à raccorder,
**caractérisé en ce que**
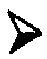 des éléments de couplage (3, 4) pour la liaison réversible avec un autre connecteur enfichable (1) présentant un corps de boîtier (2) du même type sont prévus à l'extérieur des deux côtés sur le corps de boîtier (2), et
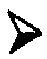 en ce que l'un des éléments de couplage est réalisé sous forme de gorge arquée (4), et l'autre élément de couplage sous forme de pion cylindrique (3).

2. Connecteur enfichable selon l'une des revendications précédentes, **caractérisé en ce que**
le corps de boîtier (2) présente un vissage de câble (40) pour une décharge de traction de câble et un étanchement.

3. Connecteur enfichable selon l'une des revendications précédentes, **caractérisé en ce que**
le corps de boîtier (2) est réalisé d'un seul tenant.

4. Connecteur enfichable selon l'une des revendications précédentes, **caractérisé en ce que**
les moyens de fixation sont réalisés à partir d'une combinaison d'ergots de fixation (5) et de pions à ressort (6).
